(19) Europäisches Patentamt
European Patent Office
Office européen des brevets

(11) **EP 4 147 660 B1**

(12) **EUROPEAN PATENT SPECIFICATION**

(45) Date of publication and mention
of the grant of the patent:
**05.03.2025 Bulletin 2025/10**

(21) Application number: **21196298.0**

(22) Date of filing: **13.09.2021**

(51) International Patent Classification (IPC):
***A61B 18/18*** *(2006.01)*

(52) Cooperative Patent Classification (CPC):
**A61B 18/1815;** A61B 2017/00128;
A61B 2018/00577; A61B 2018/00785;
A61B 2018/00904; A61B 2018/1869;
A61B 2018/1892

(54) **DUAL-MODE MICROWAVE APPLICATOR**

MIKROWELLENAPPLIKATOR MIT ZWEI BETRIEBSARTEN

APPLICATEUR DE MICRO-ONDES À DOUBLE MODE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**15.03.2023 Bulletin 2023/11**

(73) Proprietor: **Technische Universität Darmstadt
64289 Darmstadt (DE)**

(72) Inventors:
• **HESSINGER, Carolin
55130 Mainz (DE)**
• **SCHÜSSLER, Martin
63768 Hösbach (DE)**
• **PARAVICINI, Markus Tobias
55122 Mainz (DE)**
• **JAKOBY, Rolf
61191 Rosbach (DE)**

(74) Representative: **LifeTech IP
Spies & Behrndt Patentanwälte PartG mbB
Elsenheimerstraße 47a
80687 München (DE)**

(56) References cited:
US-A1- 2006 289 528    US-A1- 2007 049 917
US-A1- 2016 051 327    US-A1- 2020 289 198

**Description**

The present invention relates to a dual-mode microwave applicator and, in particular, to a coaxial microwave applicator geometry with an open end and a metal sleeve for treating malignant tissue.

BACKGROUND

The need for minimally invasive interventions such as thermal ablation therapies is continuously increasing due to rising case numbers of, e.g., liver tumor diseases. An example for the thermal ablation therapies is the microwave ablation (MWA). Its advantages lie in the much shorter treatment time as well as faster convalescence and lower risks for infections compared to a classical resection surgery.

The MWA system includes a needle-like applicator that works as an antenna at the operating frequency, a power generator and a low loss coaxial cable between those devices. Conventional MWA devices work at operating frequencies of 915 MHz or 2.45 GHz with a power typically between 60 W and 100 W for a duration about 5 to 10 min. A major challenge of MWA treatments is the accurate localization and positioning of the active part of the applicator in the center of the tumor.

As an example in the context of MWA systems, US 2006/0289528 A1 discloses a a microwave antenna suitable for cardiac ablation where an outer conductor of a coaxial structure shows a number of gaps in a tip region, and a hollow metallic cap at the tip connects the outer conductor with an inner conductor.

US 2007/0049917 A1 discloses an antenna for microwave tumor ablation, where the outer conductor of a coaxial antenna is surrounded by an insulated metallic sleeve for controlling an energy deposited into the tissue. US 2016/0051327 A1 discloses a further microwave ablation probe, wherein again a portion of the outer conductor is removed, and the inner conductor connects to a radiator structure at the tip.

During the microwave treatment of malignant tissue, it is possible to determine the position of the microwave applicator in the patient by imaging techniques such as computed tomography, ultrasound or magnetic resonance imaging (MRI). However, it is difficult to determine the exact line between malignant and healthy tissue.

Therefore, there is a need for microwave applicators that are able to determine the outline of the malignant tissue as accurately as possible, so that a treating physician can immediately determine whether the microwave applicator is in healthy tissue or in pathological tissue, based on which the treatment can be carried out.

Healthy and malignant tissues differ in their dielectric properties such as (complex-valued) permittivity, with a jump in permittivity of between 15 and 30% expected at transition. If the permittivity of the surrounding healthy tissue is measured as accurately as possible, it is possible to determine exactly when the microwave applicator passes from a healthy tissue into a malignant tissue or to where the diseased tissue extends.

However, there is an ongoing need, not only of improving the microwave ablation itself, but also to combine the treatment with a reliable monitoring.

SUMMARY OF THE INVENTION

At least some of the problems of the conventional systems are overcome by a system defined in the appended set of claims. The dependent claims refer to further advantageous realisations of the claimed subject matter.

The present invention relates to a dual-mode microwave applicator for treating tissue. The applicator comprises a control unit, a rod-shaped element, a coaxial line, and a metal sleeve. The control unit is configured to control the applicator in an ablation mode and/or a sensing mode, wherein during the ablation mode microwave radiation is applied, and during the sensing mode tumorous tissue is detected. The rod-shaped element includes a tip. The coaxial line is formed inside the rod-shaped element to relay microwaves to the tip for treatment of the tissue, wherein the coaxial line comprises an inner conductor and an outer conductor. The outer conductor includes a slot with a slot length formed at a slot distance from the tip. The metal sleeve is formed at a sleeve distance from the slot. The values of the lengths and/or distances are set as follows: the sleeve distance is about 0.55 mm and/or the slot length is about 1.75 mm and/or the slot distance is about 7.7 mm. A person skilled in the art will understand these "about"-values within certain tolerances around these values (e.g. manufacturing tolerances). Likewise, it is understood that slight deviations may achieve the same technical effect. For example, the respective tolerance region(s) may be defined by the regions of $\pm 0.5$ mm or $\pm 50\%$ (or $\pm 20\%$ or $\pm 10\%$) around the set values.

Optionally, the length/distance values are combined with at least one of the following: the metal sleeve may comprise a sleeve length of about 5.28 mm and/or a diameter of about 2.19 mm. The outer conductor may comprise an outer diameter of about 1.19mm. The outer conductor may comprise an inner diameter of about 0.97 mm. The inner conductor may comprise a diameter of about 0.3 mm. These "about" values, too, can be understood to lie within corresponding tolerance regions as defined above, e.g. manufacturing tolerances or regions of $\pm 50\%$ (or $\pm 20\%$ or $\pm 10\%$) or $\pm 0.5$ mm of the set values.

Same tolerances may be applied to other "about"-values mentioned below. However, it should be understood that the exact values provide advantages that are not necessarily achieved for other values within the tolerance regions. But they should still be covered by embodiments.

The inventors have found an unexpected strong increase in efficiency of the dual-mode applicator for the defined particular values. For this, a procedure as set out below was utilized. It was further ascertained that already small variations in a fraction of millimetres have significant effects deteriorating the desired technical effect. It is stressed that the selection of the values is connected to the particular technical effect, namely on one hand resulting in a strong increase in the efficiency of the antenna when applying microwaves to tissue and, at the same time, resulting a very high sensibility of determining complex permittivity values from reflection signals. A trade-off was made to combine both operational modes within a single device. The technical effect occurred at the defied values (or within the defined tolerance ranges) with an unexpected degree.

Optionally, the control unit is configured, in the application mode, to generate a standing wave of predetermined frequency in the coaxial line. Optionally, the control unit is configured, in the sensing mode, to use the coaxial line as receiving antenna for reflection signals from the tissue and to identify tumorous or healthy tissue. The operational frequency can be selected freely and may be in the range between 500 MHz and 10 GHz, or about 5.8 GHz (or about 915 MHz or about 2.45 GHz).

Optionally, the control unit is configured to detect tumorous tissue by detecting relative permittivity changes before and during a microwave application procedure. For example, the control unit may be configured to be switched between both modes or to alternate automatically between both modes (with controllable operation lengths for each mode) to enable a parallel treatment and sensing with the same applicator.

Optionally, the control unit is configured to provide data for visualization of the tumorous tissue around the slot (e.g. based on the data captured in the sensing mode).

Optionally, the control unit is configured to perform a calibration of the microwave applicator based on measurements on at least two predetermined environments with known permittivities and/or based on a measurement in an environment with healthy tissue. The control unit may be configured to detect healthy tissue based on previous calibrations or by assuming that the first entered tissue is presumably healthy.

Optionally, the control unit is configured to apply calibration signals to the microwave applicator which are associated with an air environment and are utilized for the calibration. Thereafter, the control unit may identify the healthy tissue to automatically finalize the calibration by a measurement on the healthy tissue.

Optionally, the applicator comprises a memory to store the permittivities of the predetermined environments and/or the air environment. The predetermined environments may be salt solutions with different salt concentrations in a range between 0.1 mol/l and 4 mol/l.

Embodiments relate also to a method for manufacturing a dual-mode microwave applicator for treating tissue. The method comprises:

- forming a coaxial line in a rod-shaped element to relay microwaves to a tip of the rod-shaped element for treatment of the tissue, wherein the coaxial line comprises an inner conductor and an outer conductor;
- forming a slot with a slot length and at a slot distance from the tip; and
- forming a metal sleeve at a sleeve distance from the slot.

The sleeve distance may be about 0.55 mm. The slot length may be about 1.75 mm. The slot distance may be about 7.7 mm. The tolerance region may again be $\pm 0.5$ mm or $\pm 50\%$ around the defined values.

In other word, according to embodiments, the microwave applicator comprises a rod-shaped treatment member in which a coaxial line is formed to transmit microwaves to a tip of the treatment member for treatment of tissue. Furthermore, the microwave applicator includes a control unit that couples to the coaxial line to deliver microwave signals including low-power signals for the sensing mode, high power signals for the ablation mode and calibration signals to the rod-shaped treatment element and to receive reflection signals. The control unit may determine, after the calibration has been performed, an edge region between the healthy tissue and the tissue to be treated. This determination can be based on a change/jump in a determined permittivity.

Further embodiments relate to a multi-objective optimization for a dual-mode microwave applicator. Dual-modality means that microwaves can be used apart from the treatment also for the monitoring of the microwave ablation intervention. The microwave ablation applicator has an optimized geometry based on sensitivity evaluations of the dual-mode applicator. Embodiments relate further to three Pareto optimal design parameter sets that are optimal in terms of applicator efficiency as well as volume and sphericity of the ablation zone. The resulting designs of the dual-mode applicator provide a suitable sensitivity to distinguish between healthy and tumorous liver tissue. Embodiment achieve an improvement of the performance over conventional applicators.

BRIEF DESCRIPTION OF THE DRAWINGS

Various embodiments of the present invention will be described in the following by way of examples only, and with respect to the accompanying drawings, in which:

Figs. 1A, 1B    depict a dual-mode microwave applicator according to embodiments of the present invention.

Fig. 2    illustrates an embodiment for a calibration procedure automatically performed by applicator.

Fig. 3A, 3B    show a three-dimension pareto plot of all parametric results (A) and under constraints of the sensitivity requirement of the dual-mode applicator (B).

Fig. 4A    shows the reflection coefficient of three pareto optimal parameter sets for the dual-mode applicator inserted into liver tissue.

Fig. 4B    shows the temperature as function of the axial direction (LA) and as function of radial direction (SA) for different parameter sets.

Fig. 4C    shows the thermal ablation zone from three-dimensional simulations of a parameter set.

DETAILED DESCRIPTION

Various examples will now be described more fully with reference to the accompanying drawings, in which some examples are illustrated.

Accordingly, while examples are capable of various modifications and alternative forms, the illustrative examples in the figures will herein be described in detail. It should be understood, however, that there is no intent to limit examples to the particular forms disclosed, but on the contrary, examples are to cover all modifications and alternatives falling within the scope of the disclosure. Like numbers refer to like or similar elements throughout the description of the figures.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

The terminology used herein is for the purpose of describing illustrative examples only and is not intended to be limiting. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, steps, operations, elements and/or components, but do not preclude the presence or addition of one or more other features, steps, operations, elements, components and/or groups thereof.

Unless otherwise defined, all terms (including technical and scientific terms) used herein have the same meaning as commonly understood by one of ordinary skill in the art to which examples belong. It will be further understood that terms, e.g., those defined in commonly used dictionaries, should be interpreted as having a meaning that is consistent with their meaning in the context of the relevant art and will not be interpreted in an idealised or overly formal sense unless expressly so defined herein.

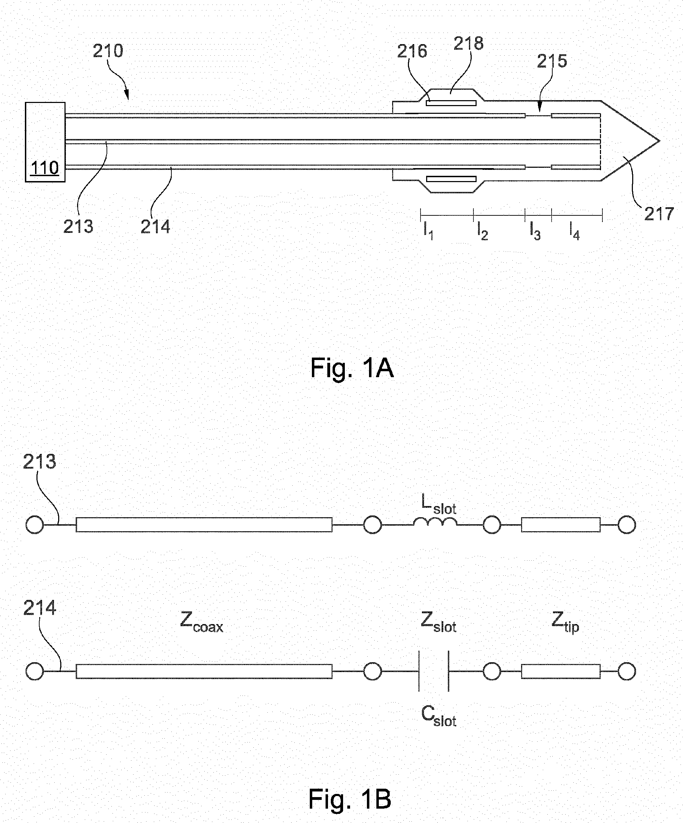

**Fig. 1A** depicts a dual-mode microwave applicator according to embodiments. The microwave applicator comprises a coaxial line having an inner conductor 213 and an outer conductor 214, with a connection to the control unit 110 being provided at one end and the coaxial line terminating in a tip 217 at an opposite end. The coaxial line is formed within a rod-shaped treatment element 210, which is suitable for treating the malignant tissue (not shown). Along the rod-shaped treatment element 210, a metal sleeve 216 and a slot (or gap) 215 are provided in the outer conductor 214. The slot 215 serves as an opening for microwave radiation from within the coaxial conductor. The metal sleeve 216 is arranged at a distance l2 from the slot 215. This suppresses the propagation of the microwave radiation away from the tip 217, thereby enabling efficient treatment of the malignant tissue 300. The metal sleeve 216 and/or slot 215 extend(s) cylindrically around the outer conductor 214. The metal sleeve 216 may be spaced or at least electrically isolated from the outer conductor 214. For example, an electrically isolating material (e.g. PTFE) may be arranged between the metallic sleeve 216 and outer electrode 214. At the tip 217, the inner and outer conductors 213, 214 are open as indicated by the dashed line. There is no electrically conducting closure or termination of the coaxial line 213, 214 at the tip 217.

Furthermore, a cover 218 is provided in the treatment area at the tip 217 to cover the area of the slot 215 and the area of the metal sleeve 216. Thus, direct contact of the metal sleeve 216 and/or outer conductor 214 with the surrounding tissue is prevented. The cover 218 may comprise, for example, a PTFE material. It may also cover the whole treatment element 210.

The axial length of the metal sleeve 216 comprises, for example, a value l1 of, for example, about 3.5 mm or 4.5 mm or 5 mm or 6 mm (or about 5.28 mm), the distance l2 comprises a value of about 0.55 mm, and the slot 115 comprises, for example, an axial extent l3 of about 1.75 mm. The distance of the gap 215 from the end portion of the coaxial cable may have, for example, a distance l4 that may be selected to be about 7.7 mm.

**Fig. 1B** shows an equivalent circuit diagram for the coaxial line along the treatment element 210. In the equivalent circuit diagram, it can be seen that the coaxial line has an impedance Zcoax, the slot section has an inductance Lslot and a capacitance Cslot resulting in an impedance Zslot. The tip 217 also corresponds to an impedance Ztip. All impedances are superimposed. When calibration signals are applied to the microwave applicator, reflections occur because the impedances will generally not be matched to the different environments. The strength of the reflections depends on the dielectric properties of the surrounding tissue or medium, so that the permittivities of the surrounding medium can be determined from the reflection coefficients.

According to embodiments, the applicator is made of a semi-rigid coaxial cable modified by one or more slots and/or metallic ground in order to achieve monopole or dipole radiation characteristics.

According to embodiments, the structure of the (dual-mode) applicator is based on a coaxial slot antenna with an open end. The particular applicator design achieves an efficient absorption of the energy in the surrounding biological material while preventing reverse currents on the outside of the applicator. For this reason, the sleeve 216 was added to the geometry.

The open-ended slot applicator is made out of a non-magnetic coaxial cable with an outer diameter of about 1.19 mm. The inner conductor and dielectric insulation between inner and outer conductor have a diameter of about 0.3 mm and about 0.97 mm, respectively. The applicator is covered with a PTFE-layer as cover 218 of about 0.2 mm thickness. Attached to this layer, the sleeve is placed with a distinct distance to the slot dsleeve (l2) and length lsleeve (l1).

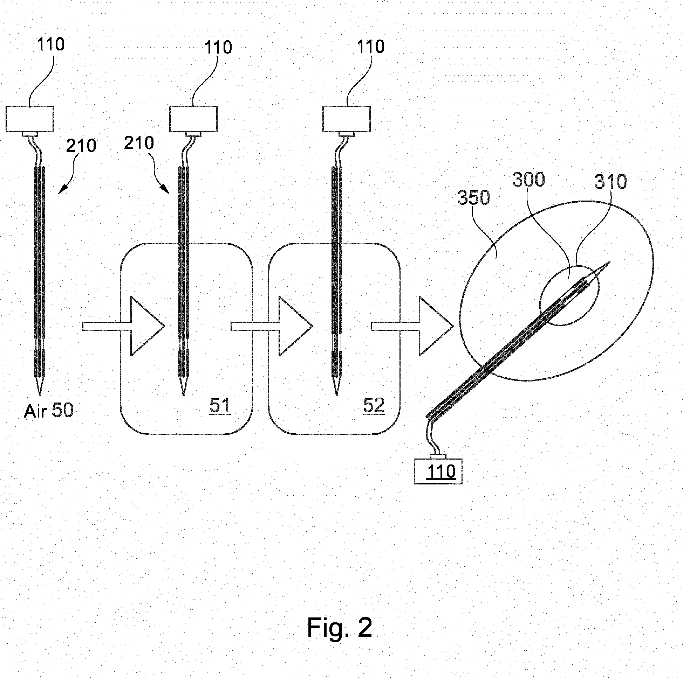

**Fig. 2** illustrates an example of the calibration procedure as automatically performed by the applicator based on predetermined environments. For example, a measurement may first be performed in an air environment 50 of the microwave applicator. Subsequently, the microwave applicator may be immersed in a first medium 51 (e.g., a liquid), calibration signals may be transmitted, and then reflection signals may be received and evaluated. Subsequently, a second medium 52 (e.g., also a liquid) can be used for calibration signals. Finally, the calibration is completed by the microwave applicator getting into contact with healthy tissue 350 to thus automatically complete the calibration.

A particular advantage of embodiments relates to the following. In the event that the malignant tissue 300 is located within healthy tissue 350, the calibration can be automatically completed as a precursor of treating the malignant tissue 300, because the microwave applicator is to be passed through the healthy tissue 350 where the measurement on the healthy tissue 350 can be performed automatically.

After the calibration, the microwave applicator can be used, for example, to treat the malignant tissue 300 in the healthy tissue 350 (e.g., for microwave ablation). For this treatment, it is particularly important that the edge 310 of the malignant tissue 300 is determined as accurately as possible so that the physician is able to determine whether the tip 217 of the microwave applicator used for treatment is in malignant tissue 300 or healthy tissue 350.

The following examples may be utilized as predetermined environments. Salt water solutions with the following salt concentrations can be used for the first and second media 51, 52: for the first liquid 51 a concentration of 0.1 mol/l and for the second liquid 52 a concentration of 2 mol/l. Optionally, other or further NaCl solutions can be selected, for example concentrations of 2.0 mol/l and/or 2.5 mol/l and/or 3.0 mol/l and/or 3.5 mol/l. For these media and for air, the permittivities $\varepsilon n$ (n=1,2, ..) are known, which are related to the associated reflection coefficients S11 via a Möbius transformation $\varepsilon n = (a*S11+b)/(c*S11+d)$, where the coefficients a, b, c, d can be found by several measurements under the condition $a*d-b*c=1$. The three unknown coefficients can be determined by three measurements on different (known) media.

However, embodiments need only two measurements on known media 51, 52.

With the measurement on healthy tissue 350, it is possible to perform the determination of the coefficients a, b, c, d up to the permittivity value of the healthy tissue 350. Since only jumps in the permittivity are to be determined for the detection of the edge 310, the knowledge of the absolute value of the permittivity of the healthy tissue 350 is not needed. Therefore, the calibration according to embodiments is easier to implement, where the healthy tissue can be automatically used as the tissue in which the tumor is embedded (its approximate location can be determined beforehand).

The efficiency of the dual-mode microwave applicator depends strongly on the selected design parameters that include the distance between the distal end of the applicator and the slot dslot (l4), the length of the slot lslot (l3), the distance between slot and sleeve dsleeve (l2), and the length of the sleeve lsleeve (l1).

Those parameters have a significant influence on the operating frequency, matching of the antenna, and the mitigation of reverse currents. In turn, these properties affect the thermal ablation characteristics that are the volume and sphericity of the lesion as well as the sensitivity to extract the dielectric properties of the surrounding medium.

The treatment monitoring tool achieves a fast and non-invasive way to observe temperature changes during the MWA when using the microwave applicator according to embodiments. The underlying method is based on the evaluation of broadband reflection signals of the microwave applicator itself in the time domain. At a boundary between tumorous and normal tissue, the dielectric discontinuity leads to a partial reflection of the electromagnetic wave propagating, from which the distance between the applicator and the edge of the tumor tissue can be calculated. This method uses a-priori knowledge of the dielectric properties of the tissue surrounding the applicator.

According to embodiments, the (dual-mode) microwave applicator incorporates a microwave sensing mode within the applicator itself to detect relative permittivity changes before and during the MWA procedure. It is designed as complementary to the magneto-resonance imaging (MRI) to realize a multimodal imaging for improving a success rate. The dual-mode concept is based on a calibration that, according to embodiments, is performed before the intervention.

According to embodiments, the design was derived by an optimization procedure based on a parametric analysis of a dual-mode microwave ablation applicator, wherein the optimization goals are minimal reflection coefficient of the antenna at the operating frequency as well as maximum volume and sphericity of the ablation zone. The optimization was constraint by the microwave sensing mode to enable the detection of a tumor 300 within the host tissue 350 (see Fig. 2).

The unidirectional electromagnetic-thermal coupled simulations were performed to obtain information about the antenna efficiency and ablation zone characteristics. In detail, the concrete design was derived based on the following simulation.

A. Simulation

For the simulations, the applicator was inserted into an exemplary material under test (MUT) block. Electromagnetic simulations were conducted in the frequency range from 0 GHz to 10 GHz with a transient solver to determine an optical antenna characteristic. The applicator was excited by a waveguide port in order to stimulate the first propagation mode within the coaxial cable. The width w, height h and length l of the MUT were chosen large enough to ensure that the electromagnetic field is either reflected or absorbed within the MUT. Thermal simulations were conducted with the thermal steady state solver. The steady state solver enables a fast calculation of the temperature distribution with a constant input temperature to analyse and compare the thermal behaviour of each applicator geometry of the parametric analysis. The temperatures were extracted along the axial and radial direction of the applicator.

Further, as also described before, electromagnetic simulations of the applicator inserted into three reference materials as calibration standards for the microwave sensing mode were conducted. As reference materials, sodium chloride (NaCl) solutions with varying concentrations and air were selected. The dielectric properties of materials containing water, such as biological tissue and sodium chloride solutions, can be described by a complex relative permittivity $\underline{\varepsilon_r} = \varepsilon'_r - j\varepsilon''_r$. The real part of the relative permittivity corresponds to the polarizability and the imaginary part is a measure of the dielectric losses of a material. The so-called dipolar polarization is a rather slow process, which can be described mathematically by the first order Debye equation

$$\varepsilon_r = \varepsilon_\infty + (\varepsilon_s - \varepsilon_\infty)/(1 + \omega\tau)\ , \qquad\qquad (1)$$

where $\varepsilon_\infty$ and $\varepsilon_s$ describe the polarizability at very high and static frequencies, $\tau$ is the duration after which the polarization of the material reaches the final value and $\omega$ describes the angular frequency. In the simulation, Debye-materials were implemented for the MUT with corresponding material parameters are summarized in the following table (at the operating frequency of the dual-mode applicator at 5.8 GHz):

| MUT | $\varepsilon_s$ | $\varepsilon\infty$ | $\tau$/ps | $\underline{\varepsilon_r}$ at 5.8 GHz |
|---|---|---|---|---|
| Liver | 49.55 | 5.32 | 11.55 | 42.893 - j15.815 |
| NaCl 0.1 mol/l | 78.1 | 5 | 9.1 | 70.7 -j21.946 |
| NaCl 2 mol/l | 59.4 | 5 | 8.13 | 55.01 - j14.82 |

As starting point for the parametric analysis of the applicator geometry, an initial design parameter set was determined. The optimization goal was to maximize the absolute value of the reflection coefficient |S11| at the operating frequency of 5.8 GHz via a trust region framework when the applicator was inserted into a liver tissue block. In order to find the optimal design parameter set in terms of maximum volume and sphericity of the ablation zone, antenna efficiency, and sensitivity of the microwave sensing mode, further parametric analysis of the applicator geometry was performed. Based on the initial design parameter set, reasonable ranges of each design parameter for the parametric analysis were selected with a step width around 0.5 mm according to the fabrication tolerances. The initial parameter set and sweep regions are summarized in following table:

| Design parameter | Initial value/mm | Sweep range | Relative change |
|---|---|---|---|
| Length sleeve $l_{sleeve}$ | 4.4 | 3.52, ..., 5.28 | ± 20 % |
| Distance sleeve $d_{sleeve}$ | 1.1 | 0.55, ..., 1.65 | ± 50 % |
| Length slot $l_{slot}$ | 1.4 | 1.05, ..., 1.75 | ± 50 % |

(continued)

| Design parameter | Initial value/mm | Sweep range | Relative change |
| --- | --- | --- | --- |
| Distance slot $d_{slot}$ | 7.1 | 5.9, ..., 8.3 | $\pm$ 18 % |

The boundaries of the simulated region were set to open (add space) for electromagnetic and thermal simulations. Further, symmetry planes of the rotational-symmetric model were set in order to reduce the simulation time. The mesh settings of the hexahedral mesh were adaptively set until the energy within the simulation cell was converged. The resulting global mesh settings with a maximum cell side of $12/\lambda$, where $\lambda$ is the smallest wavelength of the simulation. Further, local mesh properties were applied to the relatively small components of the simulation model that are the coaxial cable 213, 214, the slot 215, the teflon cover 218 and the tip 217 with a mutual edge refinement factor of 38.

B. Sensitivity Analysis

The sensitivity of the dual-mode applicator is evaluated by means of the numerical accuracy of the relative permittivity extraction at the operating frequency of about 5.8 GHz. The relation between the simulated reflection coefficient S11 and the complex relative permittivity of the MUT er is bilinear and can be formulated as

$$\varepsilon_{r} = (a^{*}S_{11} + b)/(c^{*}S_{11} + d) \,, \qquad (2)$$

where the complex coefficients a, b, c, d can be found by several measurements under the condition a*d-b*c=1 when the applicator is inserted in three reference materials with known permittivity, $\varepsilon_{r,1}$, $\varepsilon_{r,2}$, and $\varepsilon_{r,3}$ by following equation

$$\begin{pmatrix} \underline{a} \\ \underline{b} \\ \underline{c} \end{pmatrix} = \begin{pmatrix} S_{11,1} & 1 & -\underline{\varepsilon}_{r,1} \cdot S_{11,1} \\ S_{11,2} & 1 & -\underline{\varepsilon}_{r,2} \cdot S_{11,2} \\ S_{11,3} & 1 & -\underline{\varepsilon}_{r,3} \cdot S_{11,3} \end{pmatrix}^{-1} \cdot \begin{pmatrix} \underline{\varepsilon}_{r,1} \\ \underline{\varepsilon}_{r,2} \\ \underline{\varepsilon}_{r,3} \end{pmatrix}. \qquad (3)$$

As reference materials, sodium chloride solutions with a NaCl concentration of 0.1 mol/l and 2 mol/l as well as air were selected. These materials are easy and reproducible to fabricate for the NaCl solutions and well-available in hospitals as future place of action. Electromagnetic simulations for all parameter sets of the applicator inserted into each reference material were conducted and the calibration parameters for each parameter set a, b, and c were calculated according to equation (3) at the operation frequency of 5.8 GHz. In the next step, the relative permittivity of a MUT was extracted. The exemplary liver tissue was selected as MUT. Hence, the sensitivity analysis was performed by comparing the simulated and the reconstructed complex permittivity of liver tissue. The relative difference between extracted permittivity and the actual permittivity was considered as measure of the extraction accuracy and determined as follows

$$\Delta_{real} = (\varepsilon'_{ext} + \varepsilon'_{MUT})/ \varepsilon'_{MUT} \,, \qquad (4)$$

$$\Delta_{imag} = (\varepsilon''_{ext} + \varepsilon''_{MUT})/ \varepsilon''_{MUT} \,, \qquad (5)$$

For the detection of a relative dielectric contrast in the range of 20 %, the extraction error shall not exceed 10 % in order to ensure the functionality of the dual-mode applicator. This means that parameter sets within the parametric analysis with a higher extraction error cannot be considered as optimum for the following optimization.

The efficiency of the applicator and the ablation zone characteristics in terms of shape and size were optimized under constraints of the applicator's sensitivity. In the following, the objectives of the optimization are described.

C. Objectives and Optimization

The applicator efficiency is related to the computed reflection coefficient S11 in dB at the operating frequency at 5.8 GHz. The higher |S11|, the more energy is absorbed into the surrounding material. The reflection coefficients of all design parameter combinations were calculated, normalized to 50 $\Omega$.

It is desirable to achieve preferably large and spherical ablation zones. These properties can be described by the two objective metrices: volume and sphericity of the ablation zone. The heating pattern of each design parameter combination of the applicator inserted into liver tissue was simulated according to loss calculations from the electromagnetic simulations. The input power was set to 10 W at the operating frequency of 5.8 GHz. The thermal properties of liver tissue were set to a thermal conductivity of 0.469 W/K/m. Further, bioheat specific properties were considered by a blood flow coefficient of 68 000 W/K/m3 and a basal metabolic rate of 1200 W/m3. The resulting temperatures were then extracted along the axial and radial dimension of the applicator. The spatial resolution of the temperature extraction along the curves was 0.1 mm. It was assumed that temperatures above 55 °C result in tissue necrosis, and therefore, determine the axial (long axis, LA) and radial (short axis, SA) extension of the ablation zone. The volume and sphericity of the ablation zone were then calculated for each design parameter combination by

$$V = 4/3 \cdot \pi \cdot LA \cdot SA^2 \qquad\qquad (6)$$

$$S = 2 \cdot SA/LA \qquad\qquad (7)$$

The optimization of the dual-mode applicator performance comprises multiple object functions $f_i$, with $i = 1, 2, 3$, each with a distinct optimization goal. The objective functions relate to the antenna efficiency, the volume, and the sphericity of the ablation zone. The overall optimization goal was to trade-off these objectives in order to find an overall solution. The multi-objective optimization problem can be defined as

$$\text{maximize:} \quad f(x) = [f_1(x), f_2(x), f_3(x)] \qquad\qquad (8)$$

with $f1(x) = |S11(x)|$, $f2(x) = V(x)$, $f3(x) = S(x)$ and $x = x1, x2, x3, x4 \in X$, where X includes all parameter sets of the design parameters. The objective function $f_i$, with $i = 1, 2, 3$ represents the objective metrices that were introduced above. The goal of this study was to find a parameter set $x^\wedge$ that is pareto optimal in the sense that no other parameter set x exists such that $f(x) \geq f(x")$.

D. Results

The parametric analysis includes permutations of the applicator geometry. Each electromagnetic simulation needed approximately 25 min ± 5 min and thermal simulations took approximately 1 min ± 0.5 min. In the following, the pareto optimal solution of the parametric analysis for the optimization of the dual-mode applicator geometry is derived. The corresponding characterization of the pareto optimal applicator geometries are presented in terms of the applicator efficiency and ablation zone size and shape.

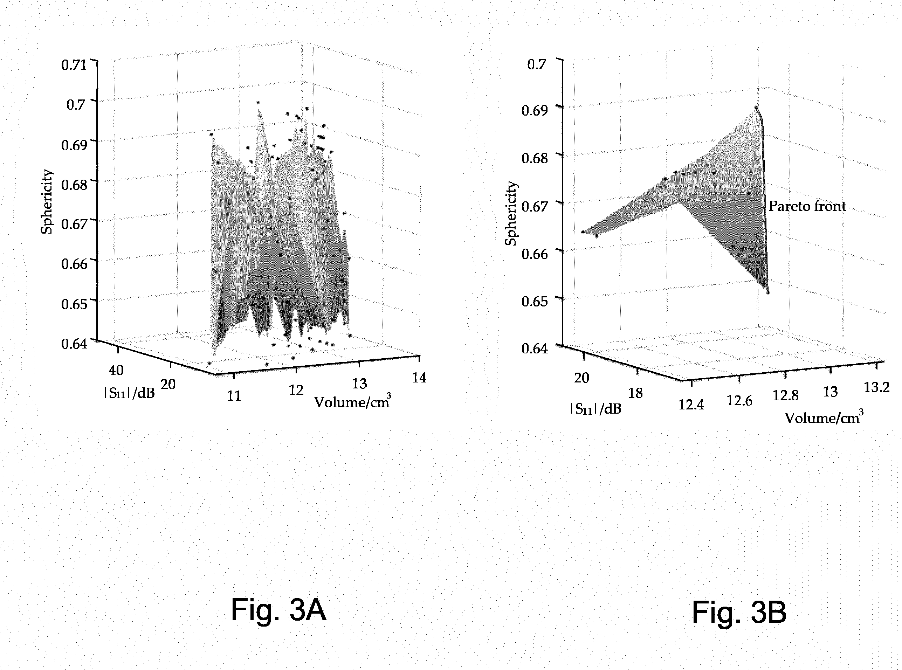

The pareto optimal solution of the parametric analysis can be graphically determined. The values of each objective function $f_i$, with $i = 1, 2, 3$ are applied to the three-dimensional pareto plot as shown in **Fig. 3A, 3B.**

The number of possible pareto optimal solutions decrease significantly by considering the sensitivity requirement from initially 225 parameter sets (Fig. 3A) to 15 possible parameter sets (Fig. 3B). The so-called pareto front describes the set of pareto optimal solutions for the case that no change of a certain objective could lead to an overall improvement of the objective function. It is drawn as a separate line on the right side in Fig. 3B. The three parameter sets $f_1$, $f_2$, f3 lie on the pareto front.

These sets are further referred to pareto optimal parameter sets. The corresponding dimensions of each design parameter, objectives and sensitivity are summarized in the following table:

| Set | Design paramete/mm | | | | Objectives | | | Sensitivity $\Delta_{real}$ |
| --- | --- | --- | --- | --- | --- | --- | --- | --- |
| | $l_{sleeve}$ | $d_{sleeve}$ | $l_{slot}$ | $d_{slot}$ | $|s_{11}|$/dB | $v$/cm$^3$ | Sphericity | |
| 1 | 5.28 | 0.55 | 1.75 | 7.7 | 18.61 | 12.94 | 0.6908 | 2.04 % |
| 2 | 4.84 | 0.55 | 1.75 | 7.7 | 18.97 | 13 | 0.6874 | 5.02% |
| 3 | 3.52 | 0.55 | 1.75 | 7.7 | 20.77 | 13.24 | 0.648 | 5.01% |

It is remarkable, that only the length of the sleeve varies for the resulting pareto optimal parameter sets. The reflection coefficient and ablation zone characteristics were determined by simulations when the applicator is inserted into liver tissue.

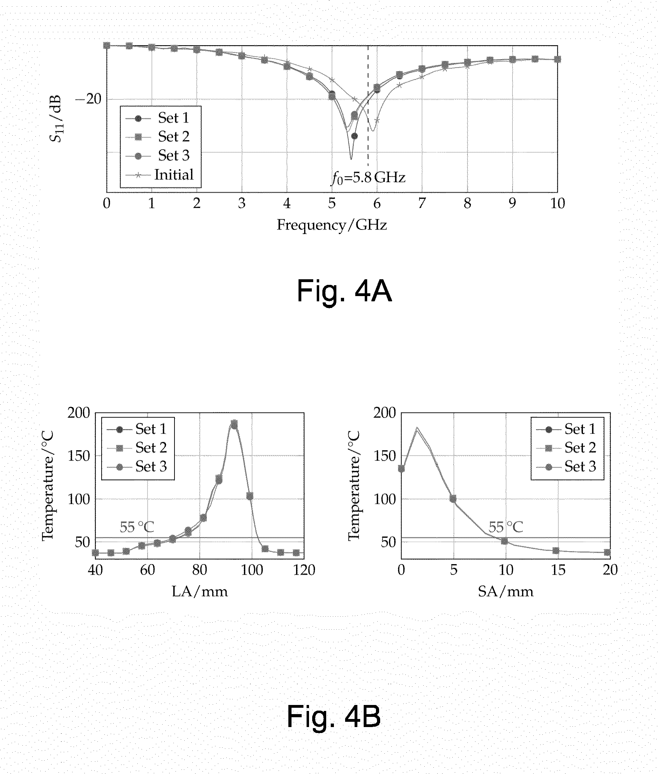

**Fig. 4A** depicts the reflection coefficient of three pareto optimal parameter sets for the dual-mode applicator inserted into liver tissue. The simulated reflection coefficients S11 are depicted as function of the frequencies from 0 GHz to 10 GHz and shown a good matching at the operating frequency of fo= 5.8 GHz below -15 dB. The pareto-optimal parameter sets are very similar. Only one from four design parameters varies that is the length of the sleeve lsleeve. With decreasing sleeve length, the reflection further decreases. As comparison to the optimized applicator design, the reflection coefficient of the initial design parameter set is shown. The initial parameter set was computed by an optimization of the reflection coefficient at the operating frequency. As a result, the corresponding reflection coefficient is S11,initial = -29.8 dB compared to S11,1= -18.61 dB, S11,2= -18.97 dB, and S11,3= -20.77 dB for parameter set 1, 2, and 3, respectively. The initial parameter set presents a better matching of the applicator to liver tissue. However, the extracted error Δinitial = 15.83 % in contrast is higher compared to the pareto optimal designs and exceeds the given sensitivity requirement.

**Fig. 4B** depicts thermal simulation results of pareto-optimal parameter sets, wherein the temperature along the axial (LA) and along the radial (SA) direction for all three sets are shown.

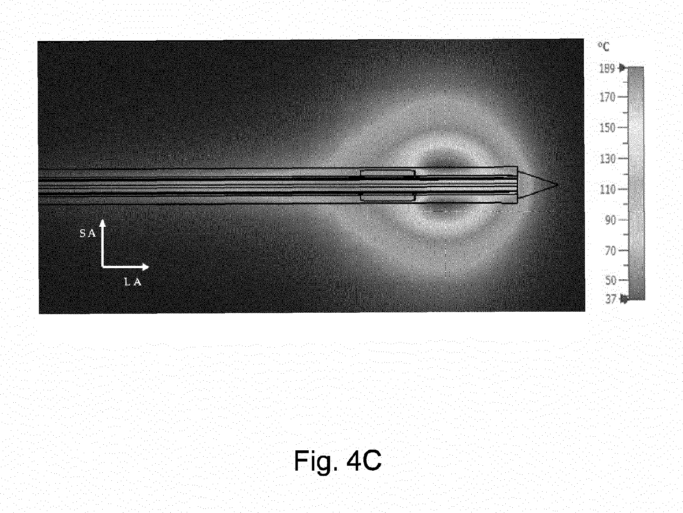

**Fig. 4C** illustrates the thermal ablation zone from three-dimensional simulations of parameter set "1". The axial temperature distribution varies only slightly between the parameter sets. With increasing sleeve length, the sphericity of the ablation zone increases and the volume decreases. Whereas, the radial temperature distribution does practically show no differences between the three parameter sets.

### E. Summary

A parametric analysis of a dual-mode microwave applicator was conducted resulting in an optimized performance of the applicator in terms of efficiency as well as size and shape of the ablation zone. Moreover, the dual-mode functionality was considered in the optimization by constraining the simulation results. The starting point of the optimization was selected by an optimization of the applicator efficiency within the exemplary liver tissue.

The multi-objective optimization procedure was based on simulations of the dual-mode applicator inserted into liver tissue as MUT. The sensitivity investigations are a key factor of the optimization procedure as it is a fundamental property to ensure a proper dual-mode functionality. The sensitivity was defined as the error between the extracted permittivity from the simulated reflection coefficient and the actual reference permittivity. Therefore, electromagnetic simulations of the applicator designs inserted into three reference materials were conducted. By doing so, the applicator performance inserted in materials with different dielectric properties than liver tissue was considered.

Regarding the applicator matching of the pareto optimal design parameter, the optimal matching frequencies of the antenna were shifted compared to the operating frequency of 5.8 GHz. Though, the matching of at least -15 dB is still good enough to create large lesions. The sensitivity analysis can be seen as a suitable method to achieve an applicator design that are not so prone to permittivity changes in the surrounding. From the resulting pareto optimal design parameter sets, a certain optimal design of the applicator with a distance between tip and slot of dslot = 7.7 mm (l4), a slot length of lslot = 1.75 mm (l3), and a distance between slot and sleeve of dsleeve = 0.55 mm (l2) was achieved.

The optimization revealed that the design is rather insensitive to changes of the sleeve length lsleeve (l1). The applicator does form a standing wave within the coaxial cable with an electric field minimum and a current density maximum at the position of the slot. That makes it less prone to reverse currents along the outer conductor of the coaxial cable.

It is understood that embodiments are based on an optimization to derive desired electromagnetic and thermal properties of the microwave applicator. Therefore, the extraction errors include numerical uncertainties.

The description and drawings merely illustrate the principles of the disclosure. It will thus be appreciated that those skilled in the art will be able to devise various arrangements that, although not explicitly described or shown herein, embody the principles of the disclosure and are included within its scope.

Furthermore, while each embodiment may stand on its own as a separate example, it is to be noted that in other embodiments the defined features can be combined differently, i.e. a particular feature described in one embodiment may also be realised in other embodiments. Such combinations are covered by the disclosure herein unless it is stated that a specific combination is not intended.

### List of reference signs

|     |     |
| --- | --- |
| 50  | air environment |
| 51, 52 | predetermined environment (e.g. NaCl solutions) |
| 110 | control unit |
| 210 | rod-shaped element |
| 213 | inner conductor |
| 214 | outer conductor |
| 215 | slot |
| 216 | metal sleeve |
| 217 | tip |
| 218 | cover |

300      tissue to be treated (malignant)
310      edge of malignant tissue
350      healthy tissue

**Claims**

1. A dual-mode microwave applicator for treating tissue, comprising:

   a control unit (110) configured to control the applicator in an ablation mode and a sensing mode, wherein during the application mode microwave radiation is applied and during the sensing mode tumorous tissue is detected;
   a rod-shaped element (210) with a tip (217);
   a coaxial line (213, 214) formed in the rod-shaped element to relay microwaves to the tip (217) for treatment of the tissue, wherein the coaxial line comprises an inner conductor (213) and an outer conductor (214), the outer conductor (214) including a slot (215) of a slot length (l3) formed at a slot distance (l4) from the tip (217); and
   a metal sleeve (216) at a sleeve distance (l2) from the slot (215),
   wherein set values are set as follows: the sleeve distance (l2) is about 0.55 mm, the slot length (l3) is about 1.75 mm, and the slot distance (l4) is about 7.7 mm, wherein a tolerance region is $\pm 0.5$ mm or $\pm 50\%$ of the set values.

2. The applicator according to claim 1, further comprising at least one of the following:

   - the metal sleeve (216) comprises a sleeve length (l1) of about 5.28 mm and/or a diameter of about 2.19 mm;
   - the outer conductor (214) comprises an outer diameter of about 1.19mm;
   - the outer conductor (214) comprises an inner diameter of about 0.97 mm;
   - the inner conductor (213) comprises a diameter of about 0.3 mm,

   wherein the tolerance region of $\pm 50\%$ or $\pm 0.5$ mm.

3. The applicator according to claim 1 or claim 2, wherein the control unit (110) is configured:

   in the ablation mode, to generate a standing wave of predetermined frequency in the coaxial line (213, 214); and
   in the sensing mode, to use the coaxial line (213, 214) as transmitting and receiving antenna for reflection signals from the tissue and to identify tumorous or healthy tissue.

4. The applicator according to claim 3, wherein the control unit (110) is configured to detect tumorous tissue by detecting relative permittivity changes before and during a microwave application procedure.

5. The applicator according to claim 4, wherein the control unit (110) is configured to provide data for visualization of the tumorous tissue around the slot (215).

6. The applicator of one of the preceding claims, wherein the control unit (110) is configured to perform a calibration of the microwave applicator based on measurements on at least two predetermined environments (50, 51, 5.2) with known permittivities ($\varepsilon$n) and/or based on a measurement in an environment with healthy tissue (350).

7. The applicator of claim 6, wherein the control unit (110) is configured to apply calibration signals for an air environment (50) to the microwave applicator before determining the healthy tissue (350), and to use the reflection signals for the air environment (51) for calibration, wherein the measurement on the healthy tissue (350) is performed automatically upon its detection by the control unit (110).

8. The applicator of claim 6 or claim 7, which further comprises a memory to store the permittivities of the predetermined environments (51, 52) and/or the air environment (50), wherein the predetermined environments (51, 52) are in particular salt solutions with different salt concentrations in a range between 0.1 mol/l and 4 mol/l.

**Patentansprüche**

1. Zweimodiger Mikrowellenapplikator zum Behandeln von Gewebe, umfassend:

   eine Steuereinheit (110), die konfiguriert ist, um den Applikator in einem Ablationsmodus und einem Erfassungsmodus zu steuern, wobei während des Anlegungsmodus Mikrowellenstrahlung angelegt wird und während des Erfassungsmodus tumoröses Gewebe erkannt wird;

ein stabförmiges Element (210) mit einer Spitze (217);

eine Koaxialleitung (213, 214), die in dem stabförmigen Element ausgebildet ist, um Mikrowellen an die Spitze (217) für die Behandlung des Gewebes weiterzuleiten, wobei die Koaxialleitung einen Innenleiter (213) und einen Außenleiter (214) umfasst, wobei der Außenleiter (214) einen Schlitz (215) mit einer Schlitzlänge ($I_3$) einschließt, der in einem Schlitzabstand (l4) von der Spitze (217) ausgebildet ist; und

eine Metallhülse (216) in einem Hülsenabstand (l2) von dem Schlitz (215),

wobei eingestellte Werte wie folgt eingestellt sind: der Hülsenabstand ($I_2$) beträgt etwa 0,55 mm, die Schlitzlänge ($I_3$) beträgt etwa 1,75 mm und der Schlitzabstand (l4) beträgt etwa 7,7 mm, wobei ein Toleranzbereich bei $\pm 0,5$ mm oder $\pm 50$ % der eingestellten Werte liegt.

2. Applikator nach Anspruch 1, ferner umfassend mindestens eines der Folgenden:

- die Metallhülse (216) umfasst eine Hülsenlänge ($I_1$) von etwa 5,28 mm und/oder einen Durchmesser von etwa 2,19 mm;
- der Außenleiter (214) umfasst einen Außendurchmesser von etwa 1,19 mm;
- der Außenleiter (214) umfasst einen Innendurchmesser von etwa 0,97 mm;
- der Innenleiter (213) umfasst einen Durchmesser von etwa 0,3 mm, wobei der Toleranzbereich $\pm 50$ % oder $\pm 0,5$ mm beträgt.

3. Applikator nach Anspruch 1 oder 2, wobei die Steuereinheit (110) konfiguriert ist:

in dem Ablationsmodus, um eine stehende Welle einer vorbestimmten Frequenz in der Koaxialleitung (213, 214) zu erzeugen; und

in dem Erfassungsmodus, um die Koaxialleitung (213, 214) als Sende- und Empfangsantenne für Reflektionssignale aus dem Gewebe zu verwenden und tumoröses oder gesundes Gewebe zu identifizieren.

4. Applikator nach Anspruch 3, wobei die Steuereinheit (110) konfiguriert ist, um tumoröses Gewebe zu erkennen, durch Erkennen von Änderungen einer relativen Permittivität vor und während einer Mikrowellenanlegungsprozedur.

5. Applikator nach Anspruch 4, wobei die Steuereinheit (110) konfiguriert ist, um Daten für eine Visualisierung des tumorösen Gewebes um den Schlitz (215) herum bereitzustellen.

6. Applikator nach einem der vorstehenden Ansprüche, wobei die Steuereinheit (110) konfiguriert ist, um eine Kalibrierung des Mikrowellenapplikators basierend auf Messungen in mindestens zwei vorbestimmten Umgebungen (50, 51, 52) mit bekannten Permittivitäten ($\varepsilon$n) und/oder basierend auf einer Messung in einer Umgebung mit gesundem Gewebe (350) durchzuführen.

7. Applikator nach Anspruch 6, wobei die Steuereinheit (110) konfiguriert ist, um vor dem Bestimmen des gesunden Gewebes (350) Kalibriersignale für eine Luftumgebung (50) an den Mikrowellenapplikator anzulegen und die Reflektionssignale für die Luftumgebung (51) für die Kalibrierung zu verwenden, wobei die Messung an dem gesunden Gewebe (350) nach dessen Erkennung durch die Steuereinheit (110) automatisch durchgeführt wird.

8. Applikator nach Anspruch 6 oder 7, der ferner einen Speicher umfasst, um die Permittivitäten der vorbestimmten Umgebungen (51, 52) und/oder der Luftumgebung (50) zu speichern, wobei die vorbestimmten Umgebungen (51, 52) insbesondere Salzlösungen mit unterschiedlichen Salzkonzentrationen in einem Bereich zwischen 0,1 mol/l und 4 mol/l sind.

**Revendications**

1. Applicateur de micro-ondes à double mode permettant de traiter un tissu, comprenant :

une unité de commande (110) configurée pour commander l'applicateur dans un mode d'ablation et un mode de captage, dans lequel pendant le mode d'application un rayonnement de micro-ondes est appliqué et pendant le mode de captage un tissu tumoral est détecté ;

un élément en forme de tige (210) avec une pointe (217) ;

une ligne coaxiale (213, 214) formée dans l'élément en forme de tige pour relayer des micro-ondes vers la pointe (217) pour le traitement du tissu, dans lequel la ligne coaxiale comprend un conducteur interne (213) et un conducteur externe (214), le conducteur externe (214) comportant une fente (215) d'une longueur de fente (l3) formée à une distance de fente (l4) par rapport à la pointe (217) ; et

un manchon métallique (216) à une distance de manchon (l2) par rapport à la fente (215),

dans lequel des valeurs réglées sont réglées comme suit : la distance de manchon (l2) est d'environ 0,55 mm, la longueur de fente (l3) est d'environ 1,75 mm, et la distance de fente (l4) est d'environ 7,7 mm, dans lequel une région de tolérance est de $\pm 0,5$ mm ou $\pm 50$ % des valeurs réglées.

2. Applicateur selon la revendication 1, comprenant en outre au moins l'un de ce qui suit :

- le manchon métallique (216) comprend une longueur de manchon ($l_1$) d'environ 5,28 mm et/ou un diamètre d'environ 2,19 mm ;
- le conducteur externe (214) comprend un diamètre externe d'environ 1,19 mm ;
- le conducteur externe (214) comprend un diamètre interne d'environ 0,97 mm ;
- le conducteur interne (213) comprend un diamètre d'environ 0,3 mm,

dans lequel la région de tolérance est de $\pm 50$ % ou $\pm 0,5$ mm.

3. Applicateur selon la revendication 1 ou la revendication 2, dans lequel l'unité de commande (110) est configurée :

dans le mode d'ablation, pour générer une onde stationnaire de fréquence prédéterminée dans la ligne coaxiale (213, 214) ; et

dans le mode de captage, pour utiliser la ligne coaxiale (213, 214) en guise d'antenne de transmission et de réception pour des signaux de réflexion provenant du tissu et pour identifier un tissu tumoral ou sain.

4. Applicateur selon la revendication 3, dans lequel l'unité de commande (110) est configurée pour détecter un tissu tumoral en détectant des changements de permittivité relative avant et pendant une procédure d'application de micro-ondes.

5. Applicateur selon la revendication 4, dans lequel l'unité de commande (110) est configurée pour fournir des données pour visualisation du tissu tumoral autour de la fente (215).

6. Applicateur selon l'une des revendications précédentes, dans lequel l'unité de commande (110) est configurée pour mettre en œuvre un étalonnage de l'applicateur de micro-ondes en fonction de mesures sur au moins deux environnements prédéterminés (50, 51, 52) avec des permittivités ($\varepsilon$n) connues et/ou en fonction d'une mesure dans un environnement avec un tissu sain (350).

7. Applicateur selon la revendication 6, dans lequel l'unité de commande (110) est configurée pour appliquer des signaux d'étalonnage pour un environnement d'air (50) à l'applicateur de micro-ondes avant détermination du tissu sain (350), et pour utiliser les signaux de réflexion pour l'environnement d'air (51) pour un étalonnage, dans lequel la mesure sur le tissu sain (350) est mise en œuvre automatiquement lors de sa détection par l'unité de commande (110).

8. Applicateur selon la revendication 6 ou la revendication 7, qui comprend en outre une mémoire pour stocker les permittivités des environnements prédéterminés (51, 52) et/ou de l'environnement d'air (50), dans lequel les environnements prédéterminés (51, 52) sont en particulier des solutions de sel avec différentes concentrations en sel dans une plage entre 0,1 mol/l et 4 mol/l.

## Fig. 1A

## Fig. 1B

Fig. 2

Fig. 3A

Fig. 3B

Fig. 4A

Fig. 4B

Fig. 4C

**EP 4 147 660 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060289528 A1
- US 20070049917 A1
- US 20160051327 A1